# EUROPEAN PATENT APPLICATION

(11) **EP 1 709 943 A2**
(43) Date of publication of application: **11.10.2006**
(21) Application number: 05109766.5
(22) Date of filing: 20.10.2005
(51) Int. Cl.: A61F 2/36, A61F 2/30, A61L 27/02

(54) **Artificial joint system**

(30) Priority: 07.04.2005 KR 2005028824
(71) Applicant: Korea Advanced Institute of Science and Technology (KAIST), Yusong-gu Daejon 305-701 (KR)
(72) Inventor: YOON, Yong-San, 305-707, Daejon (KR)
(74) Representative: Michalski, Stefan

(57) **Abstract**

An artificial joint apparatus (100) used as an artificial joint inserted into a bone (200) is provided. The artificial joint apparatus (100) includes: a body having a stem (110) having a shape substantially corresponding to a space within the bone, into which the stem (110) is inserted, and a head (112) formed integrally with the stem (110); and a jacket (120) made of a plastic material and surrounding an outer surface of the stem (110) of the body. The jacket (120) is constructed with an outer layer containing a bone-friendly material (130) and an inner layer mainly made of a plastic material. Since the plastic jacket (120) of an artificial joint apparatus (100) has a bone-friendly material (130), it is possible to promote a growth of a bone contacted thereto. In addition, the bone is grown into a space where the bone-friendly material (130) is dissolved, so that the jacket (120) and the bone can be securely adhered. Since the bone-friendly material (130) also exists in an inner portion of the jacket (120) it is possible to continuously accelerate a bone in-growth.

## Description

### CROSS-REFERENCE TO RELATED APPLICATIONS

This application claims the benefit of Korean Patent Application No. 10-2005-0028824, filed on April 7, 2005, in the Korean Intellectual Property Office, the disclosure of which is incorporated herein in its entirety by reference.

### FIELD OF THE INVENTION

The present invention relates to an artificial joint apparatus, and more particularly, to an artificial joint apparatus having a jacket made of a plastic material containing a bone-friendly material.

### BACKGROUND OF THE INVENTION

An artificial joint, for example, an artificial joint used for an artificial hip joint surgery has an acetabular portion which is inserted into a pelvis as a substitute for an acetabulum and a femoral portion which is inserted into a femur as a substitute for a head. The artificial joint is mainly made of a material such as metal, ceramic, and plastic.

The femoral portion of the artificial joint, which is inserted into the femur, is generally consist of portion corresponding to the head and a stem which is a portion inserted into the femur. In the surgery, a space is formed by partially digging a marrow cavity of the femur, the space is filled with a suitable amount of cement, and the stem of the artificial joint is inserted into the space. The cement has a function of adhering the artificial joint to the femur. A surface of the stem of the artificial joint may be coated with cement made of the same material as the cement filling the space in order that the stem can be securely adhered to the femur. However, in case of adhering the artificial joint by using the cement, since the adhesive force is relatively weak, there is a problem in that lifetime of the artificial joint is not long.

As another kind of an artificial joint for which a cement is not used, there is an artificial joint of which stem is coated with a porous plastic jacket. The stem where a porous plastic layer is formed on a surface thereof is inserted into a femur, and the porous plastic surface is physiologically bonded to the stem to be fixed to the femur while the femur is gradually grown. However, in case of such artificial joint, stress is concentrated on a bonding surface between the femur and the plastic layer, so that the bonding surface may be partially separated to form a gap. As a result, there is a problem in that a contaminant may penetrate into the gap to experdite osteolysis of the femur. In addition, since the plastic has no function of inducing growth of bone, there is another problem in that too much time is required to fix the artificial joint to the femur.

An artificial joint of which stem is provided with a porous plastic jacket is disclosed in Korean Patent Publication No. 10-2003-62812. A surface of the plastic jacket of the artificial joint is coated with a bonding promoter. As the bonding promoter, hydroxyapatite or tricalcium phosphate is used. The bonding promoter accelerates bonding of the plastic jacket to the femur, so that the artificial joint can be rapidly fixed. However, similar to the aforementioned conventional technique, since the plastic jacket is bonded to the femur by using a porosity of the plastic material, the bonding promoter of the plastic jacket stem may be separated due to a repeatedly applied weight, so that the bonding surface between the jacket and the femur may be separated. Therefore, there is a problem in that a contaminant penetrates, so that the osteolysis of the femur may occur.

### SUMMARY OF THE INVENTION

In order to solve the aforementioned problems, an object of the present invention is to prolong lifetime of an artificial joint by continuously increasing an adhesive strength of a plastic jacket and a femur without an additional adhesive.

In accordance with an aspect of the present invention, there is provided an artificial joint apparatus used as an artificial joint inserted into a bone, comprises: a body having a stem having a shape substantially corresponding to a space within the bone, which the stem is inserted into, and a head formed integrally with the stem; and a jacket made of a plastic material and surrounding an outer surface of the stem of the body, wherein the jacket is constructed with an outer layer containing a bone-friendly material and an inner layer made of a plastic material.

The bone-friendly material may be an active bone-friendly material and include hydroxyapatite, various kinds of calcium phosphates including tricalcium phosphate or a bone powder. The jacket may further include human-harmless ceramic or metal particles as a passive bone-friendly material.

A material for the stem of the body may be a metal selected from a group consisting of stainless steel, titanium, and an alloy thereof. In addition, the material for the stem of the body may be a reinforced ceramic or a carbon fiber.

### BRIEF DESCRIPTION OF THE DRAWINGS

The above and other features and advantages of the present invention will become more apparent by describing in detail exemplary embodiments thereof with reference to the attached drawings in which:
FIG. 1 is a schematic view showing an artificial joint apparatus in accordance with an embodiment of the present invention;
FIG. 2 is a perspective view of a plastic jacket of an artificial joint apparatus in accordance with an embodiment of the present invention;
FIG. 3 is an enlarged cross sectional view of the plastic jacket shown in FIG. 2; and
FIG. 4 is a view of a state where an artificial joint apparatus in accordance with an embodiment of the present invention is disposed to a femur.

### DETAILED DESCRIPTION OF THE PREFERRED EMBODIMENT

Now, preferred embodiments of the present invention will be described in detail with reference to the accompanying drawings.

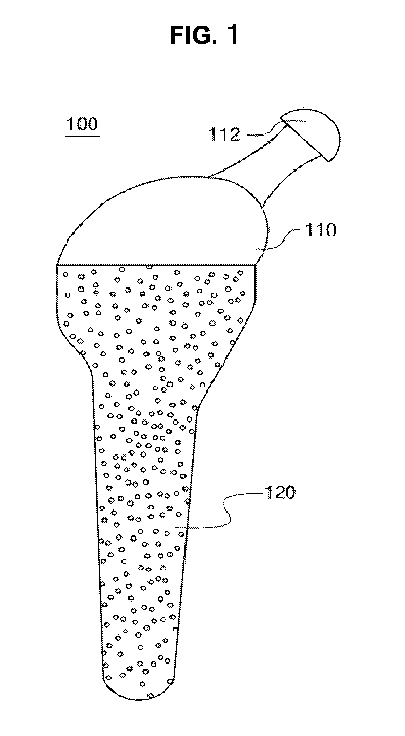

FIG. 1 is a schematic view showing an artificial joint apparatus in accordance with an embodiment of the present invention.

As shown in FIG. 1, an artificial joint apparatus 100 includes a body having a stem 110 inserted into a bone which is to be operated and a femoral head 112 having a shape of a head and a jacket 120 surrounding an outer surface of the stem 110. The stem 110 is made of a metal material and has a shape substantially corresponding to an inserting space previously provided to the bone which is to be operated. The jacket 120 is made of a plastic material and has particle-like bone-friendly materials.

The stem 110 provided to the body of the artificial joint is a portion which is inserted into a space provided to an inner portion of the bone which is to be operated and has a thin and long shape. The space into which the stem 110 is inserted is formed by deeply digging a marrow cavity of the bone 200 which is to be operated. The stem 110 is made of a metal material, for example, a cobalt-chromium alloy having a high strength. Preferably, the stem 110 may be made of stainless steel, titanium, or an alloy thereof. These materials are known as human-harmless material and inexpensive in comparison to a cobalt-chromium alloy, so that cost of the artificial joint surgery can be reduced. The material for the stem is not limited to these metal materials, but a non-metal material such as a ceramic material and a carbon fiber may be used. The head 112 having a shape of a femoral head is integrally connected to the stem 110. The shape of the head is formed corresponding to a shape of an artificial acetabulum cup. In the embodiment, the head has a shape of a semi-sphere. The stem 110 and the head 112 are connected to each other via a neck portion.

The jacket 120 surrounding the stem is made of a plastic material. When the artificial joint is inserted into the bone which is to be operated, the outer surface of the jacket 120 directly contacts an inner surface of the bone. The jacket 120 has particle-like bone-friendly materials. As the bone-friendly material, hydroxyapatite, tricalcium phosphate, a bone powder of an animal, or any kinds of calcium phosphate having a characteristic of accelerating growth of bone is used. In addition, auxiliary particles such as the ceramic particles and metal particles, which do not actively accelerate growth of the bone but is verified to have a bone in-growth characteristic and can rapidly and securely fix the jacket 120 to the bone by increasing abrasion and contact area of the plastic jacket 120 may also be used as the bone-friendly material. Preferably, these particles exist in the inner portion of the jacket as well as on the surface of the jacket, as described later.

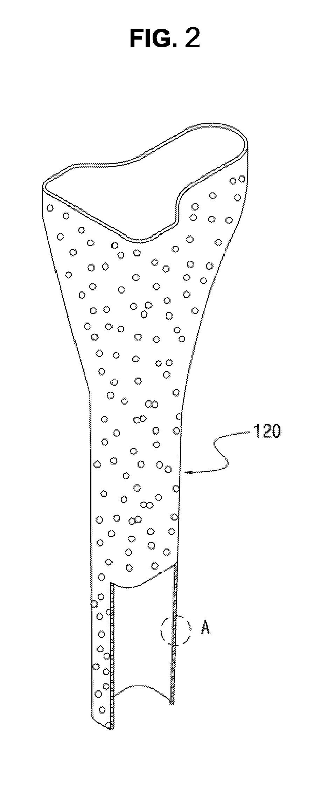

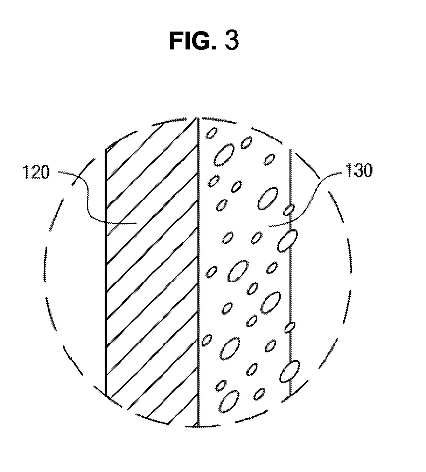

FIG. 2 shows a partially cut-away cross section of the jacket 120, and FIG. 3 is an enlarged view of a portion indicated by circle A of FIG. 2. As shown in the figures, the plastic jacket 120 has a shape of a pouch, of which upper portion opens. The bone-friendly material particles 130 provided to the plastic jacket exist on the surface and in the inner portion of the jacket. As time elapses, the bone-friendly material particles are dissolved and absorbed into the bone. At this time, since the bone is grown into the space where the bone-friendly material previously exists, the jacket can be securely fixed to the bone. In addition, since the bone-friendly material particles are distributed not only on the surface of the jacket 120 but also in the inner portion of the jacket 120, the bone in-growth can be continuously accelerated even after the bone-friendly material particles distributed on the surface of the jacket absorbed into the bone.

Preferably, a large amount of the bone-friendly material particles 130 are distributed in a portion close to the outer surface of the jacket, and a relatively small amount thereof are distributed in a portion close to the inner surface the jacket. If the particles are distributed in such a manner, a strength of the plastic jacket 120 can be suitably maintained. For example, an inner layer of the jacket is firstly formed with a plastic material which does not contain the bone-friendly material, and after that, an outer layer of the jacket is formed with the mixed plastic material, so that the plastic jacket 120 in which the bone-friendly material 130 is disturbed as shown in FIG. 3 can be manufactured.

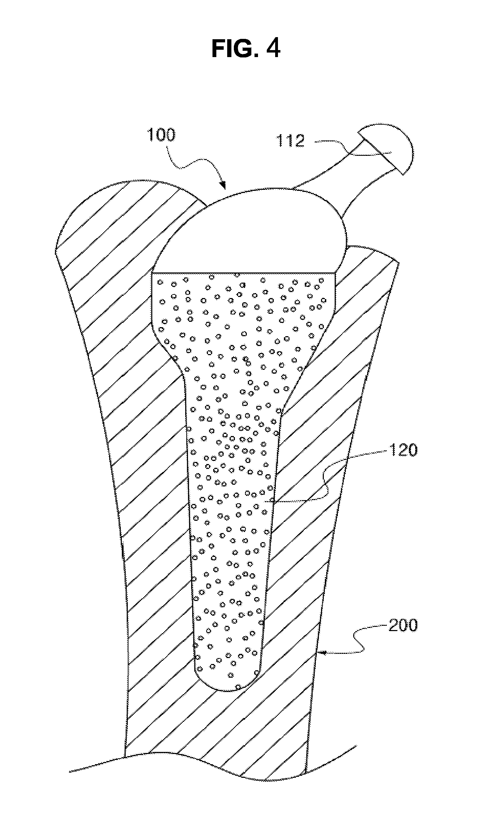

FIG. 4 shows a usage of an artificial joint in accordance with the present invention. A marrow cavity is previously removed from a bone 200 which is to be operated, so that a space into which the artificial joint 100 is inserted can be prepared. An artificial joint is inserted into the prepared space. Any adhesive such as a cement is not used. An inserted portion is mainly a stem 100 surrounded by a plastic jacket 120, and a head 112 having a function of a head protrudes outward the bone. Since a bone-friendly materials are prepared on a surface and in an inner portion of the plastic jacket 120, the artificial joint and the bone can be securely adhered to each other without any cement.

While the present invention has been particularly shown and described with reference to exemplary embodiments thereof, it will be understood by those of ordinary skill in the art that various changes in form and details may be made therein without departing from the spirit and scope of the present invention as defined by the following claims.

According to the present invention, a plastic jacket of an artificial joint apparatus has a bone-friendly material, so that it is possible to accelerate a growth of a bone contacted thereto. The bone is grown into a space where the bone-friendly material is dissolved, so that the jacket and the bone can be securely adhered. In addition, the bone-friendly material also exists in an inner portion of the jacket, so that it is possible to continuously accelerate a bone in-growth.

## Claims

1. An artificial joint apparatus used as an artificial joint inserted into a bone, comprising:
a body having a stem made of a metal and having a shape corresponding to a space within the bone, into which the stem is inserted, and a head formed integrally with the stem; and
a jacket made of a plastic material and surrounding an outer surface of the stem of the body,
wherein the jacket has a bone-friendly material, and,
wherein the bone-friendly material is distributed on an outer surface of the jacket and in an inner portion of the jacket.

2. The artificial joint apparatus in accordance with claim 1, wherein the bone-friendly material is distributed on the outer surface of the jacket and in an inner region close to the outer surface, and wherein the bone-friendly material is not distributed in an inner region close to an inner surface of the jacket.

3. The artificial joint apparatus in accordance with claim 1, wherein the bone-friendly material is one selected from a group consisting of hydroxyapatite, tricalcium phosphate, and a bone powder.

4. The artificial joint apparatus in accordance with claim 1, wherein the jacket further has human-harmless ceramic or metal particles.

5. The artificial joint apparatus in accordance with claim 1, wherein the stem of the body is made of a metal selected from a group containing stainless steel, titanium, and an alloy thereof.

6. The artificial joint apparatus in accordance with claim 1, wherein the stem of the body is made of a reinforced ceramic material or a carbon fiber.
